# EUROPEAN PATENT SPECIFICATION

(11) **EP 0 569 043 B1**
(45) Date of publication and mention of the grant of the patent: **08.01.1997**
(21) Application number: 93108543.5
(22) Date of filing: 09.08.1989
(51) Int. Cl.: C07D 519/04, A61K 31/395

(54) **Method for the preparation of 3',4'-anhydrovinblastine**
Verfahren zur Herstellung von 3',4'-Anhydrovinblastin
Méthode de préparation de 3',4'-anhydrovinblastine

(30) Priority: 11.08.1988 JP 198897/88; 11.08.1988 JP 198898/88
(43) Date of publication of application: 10.11.1993
(62) Divisional of application: 89308089.5
(73) Proprietor: MITSUI PETROCHEMICAL INDUSTRIES, LTD., Tokyo 100 (JP)
(72) Inventor: Tan, Hiroaki, c/o Mitsui Petrochem. Ind. Ltd., Kuga-gun, Yamaguchi-ken (JP); Sakamoto, Naoya, c/o Mitsui Petrochem. Ind. Ltd., Kuga-gun, Yamaguchi-ken (JP); Hata, Eiichirou, c/o Mitsui Petrochem. Ind. Ltd., Kuga-gun, Yamaguchi-ken (JP); Ishitoku, Takeshi, c/o Mitsui Petrochem. Ind. Ltd., Kuga-gun, Yamaguchi-ken (JP); Kihara, Noriaki, c/o Mitsui Petrochem. Ind. Ltd., Kuga-gun, Yamaguchi-ken (JP)
(74) Representative: Cresswell, Thomas Anthony

(56) References cited:
- WO-A-88/02002
- TETRAHEDRON, (INCL. TETRAHEDRON REPORTS) vol. 44, no. 2, 1988, OXFORD GB pages 325 - 331 J. VUKOVIC ET AL. 'Production of 3',4'-anhydrovinblastine: a unique chemical synthesis'

## Description

This invention relates to method for producing 3',4'-anhydrovinblastine. 3'4'-Anhydrovinblastine is useful as an antineoplastic drug.

Dimeric alkaloids such as 3',4'-anhydrovinblastine are known to possess an antineoplastic activity (US-A-4,029,663). Known methods of obtaining these compounds include methods which consist of extracting the compounds from a plant of the genus Catharanthus, having the scientific name Catharanthus roseous (alias Vinca rosea), and methods which resort to chemical synthesis using catharanthine and vindoline derived from a plant as starting materials.

The extraction methods, however, entail difficulties in separation and purification of the dimeric alkaloids because the plants used have a very low dimeric alkaloid content and also because analogous compounds are additionally present in the plants.

Among the methods resorting to chemical synthesis, the method which comprises oxidizing catharanthine with a peracid, acylating the resultant N-oxide, reacting the acylated product with vindoline and reducing the reaction product with NaBH₄ (US-A-4,144,237) is known in the art. This method, however, attains isolation of 3',4'-anhydrovinblastine only in a low yield of 41% and simultaneously gives rise to a 10% yield of isomers as a by-product. Thus, this method entails difficulties in separation and purification of the dimeric alkaloids.

The inventors, mindful of commercialized production of dimeric alkaloids, have found a special interest in the method which comprises effecting coupling of catharanthine with vindoline in the presence of Fe³⁺ and then reacting the product of coupling with a reducing agent or a hydride source. They tried reacting the product of coupling with various reducing agents or hydride sources, to find that this method is not particularly effective in improving the yield of the dimeric alkaloids.

The inventors continued a study with a view to improving the yield of dimeric alkaloids, and acquired the unexpected and useful novel knowledge that the removing or inactivating the Fe³⁺ prior to the addition of the reducing agent, the yield of 3',4'-anhydrovinblastine is notably improved. Accordingly, the invention provides a method for producing 3',4'-anhydrovinblastine, which comprises reacting catharanthine with vindoline in the presence of Fe³⁺, removing or inactivating the Fe³⁺, by addition of any compound selected from aqueous ammonia, a lower fatty acid, a salt thereof, a dicarboxylic acid, a salt thereof, a ketocarboxylic acid, a salt thereof, a hydroxycarboxylic acid, a salt thereof and a fluoride, and subsequently reacting the reaction product with a reducing agent.

In the working of this invention, the reaction of catharanthine with vindoline in the presence of Fe³⁺ can be carried out as conventionally practised. Generally, this reaction brings about desirable results when it is carried out in an atmosphere of nitrogen or under a current of nitrogen.

The removal of Fe³⁺ may be attained by adding aqueous ammonia to the reaction mixture after completion of the coupling reaction thereby inducing precipitation of Fe³⁺, and removing the precipitate from the reaction system by a conventional solid-liquid separation technique, such as filtration or centrifugal separation.

The term "inactivation of Fe³⁺" refers to any treatment which is capable of eliminating or diminishing the adverse effects or impeditive actions of Fe³⁺ otherwise manifested in the subsequent steps. The treatment for the inactivation of Fe³⁺ therefore embraces treatments capable of inhibiting the coordination of iron to useful components in the subsequent steps and actions capable of inhibiting the oxidation by iron. Examples of methods which may be used include a method which comprises adding an iron ligand to the reaction system after completion of the coupling reaction thereby inactivating the Fe³⁺ and precluding the adverse effects of impeditive actions of Fe³⁺, a method which effects inactivation by reducing the Fe³⁺ to metallic iron or Fe²⁺, and a method which comprises producing different species of iron by the action of different species of ligands and causing the resultant complexes to be coprecipitated as complex salts or converted into nontoxic solubles. In a preferred method, the precipitate of Fe³⁺ with the aqueous ammonia is either allowed to remain wholly in the reaction system or partly removed, allowing the remainder to remain in the reaction system.

The method for inactivating Fe³⁺ by the addition of an iron ligand is a method which comprises adding at least one of the followning compounds capable of coordinating the Fe³⁺:
Lower fatty acids, for example formic acid, acetic acid, propionic acid, butyric acid, valeric acid, trimethyl acetic acid, caproic acid, enanthic acid, and caprylic acid.
Dicarboxylic acids, for example aliphatic dicarboxylic acids (oxalic acid, malonic acid, succinic acid, glutaric acid, adipic acid, maleic acid, fumaric acid, and pimelic acid) and aromatic dicarboxylic acids (phthalic acid, isophtalic acid, and terephthalic acid).
Ketocarboxylic acids, for example pyruvic acid.
Hydroxycarboxylic acids, for example aliphatic hydroxycarboxylic acids (glycolic acid, lactic acid, hydroxyacrylic acid, glyceric acid, malic acid, tartaric acid, succinic acid, and citric acid) and aromatic oxycarboxylic acids (salicylic acid, oxybenzoic acid, and gallic acid), and
Salts of the foregoing compounds.
Fluorides, for example sodium fluoride and potassium fluoride.

Such an iron ligand is suitably added in an amount of 0.5 to 200 mol, preferably 1 to 10 mol, per mol of Fe³⁺.

After the inactivation of Fe³⁺ is carried out as described above, a reducing agent is added to the reaction system to give the target compound.

When the precipitate of Fe³⁺ is produced by the treatment of inactivation, the reduction may be performed on the reaction liquid which remains after removal of the precipitate, such as the filtrate. The precipitate which is removed from the reaction system is washed with an organic solvent. The washings are combined with the reaction liquid such as the filtrate or the supernatant, concentrated under a vacuum to expel the organic solvent, admixed with water, and then reduced with a reducing agent such as NaBH₄. Alternatively, the mixture of the washings with the reaction liquid, without being concentrated under a vacuum, may be adjusted to a pH value of less than 6, subjected to solid-liquid separation, and the water layer reduced by the addition of a reducing agent such as NaBH₄. The precipitate produced in consequence of the treatment of inactivation is not necessarily removed from the reaction system. When the treatment of inactivation is carried out in a liquid state, the reaction mixture is subjected to extraction of the water layer with an organic solvent. The organic layer consequently obtained is concentrated under a vacuum, admixed with water, and then reduced.

Examples of the reducing agents which may be used include the following compounds.
Hydrides: sodium borohydride, potassium borohydride, lithium borohydride, and sodium cyanoborohydride.
Metals: zinc, iron, tin, aluminum, and magnesium.

When such a reducing agent as mentioned above exhibits an excessively high reducing power, the excessive reducing power may suitably be diminished by decreasing the amount of the reducing agent or having the reducing agent itself suitably modified or masked.

Though the coupling reaction may be performed in an atmosphere of air, it brings about better results when it is carried out in an atmosphere of nitrogen or under a current of nitrogen.

The 3',4'-anhydrovinblastine which is formed by the method described above can be isolated by extraction with an organic solvent.

By this method, since the target compound is obtained in a very high purity, it can easily be crystallized. Thus, it can be thoroughly recovered by recrystallization without requiring any of the preliminary isolation and purification treatments by the use of a column, which are an indispensable requirement of the conventional method. Since the separation of the product is easy, the present method is highly suitable for commercial production.

The present invention will now be illustrated by Examples.

### Example 1

In a reaction vessel having an inner volume of 500 ml, 180 ml of water was placed, swept with N₂ gas and, at the same time, cooled with ice. To this water, a solution of 184 mg (480 µmol) of a half-sulfate of catharanthine in 20 ml of water and a solution of 220 mg (480 µmol) of vindoline in 20 ml of water and 0.4 ml of 2N-HCl were added. To the resultant mixture, a solution of 8.1 g of FeCl₃.6H₂O in 50 ml of water was added to induce reaction. Under a continuous forced introduction of N₂, the reaction mixture was stirred for three hours under ice-cooling, and a solution of 12.2 g of NaOAc in 50 ml of water was added. The resultant mixture and 227 mg of NaBH₄ added in a solid state thereto were stirred for 30 minutes. The reaction mixture was extracted four times with 100 ml of ethyl acetate. The organic layer consequently separated was dried over anhydrous sodium sulfate and then concentrated under a vacuum. The residue of the concentration was dissolved in 30 ml of chloroform and then washed with 50 ml of a saturated aqueous solution of NaHCO₃. The organic layer was dried over anhydrous sodium sulfate and then concentrated. The residue of concentration, on mixing with 2 ml of methanol, precipitated as crystals. When the crystals were separated by filtration, there were obtained 304 mg of colorless crystals of 3',4'-anhydrovinblastine having a melting point of 200°C (decomposition). The yield of the produce was 80%. This compound gave signals at 3.89 (3H, S) and 3.54 (3H, S) in the ¹H-nmr(CDC1₃) spectrum. Thus, they were identified as 3',4'-anhydrovinblastine.

### Example 2

In a reaction vessel having an inner volume of 50 ml, 10 ml of water was placed, kept cooled with ice and, at the same time, bubbled with nitrogen for 30 minutes. To this water, 200 µl (2.39 µmol) of an aqueous 11.9 mM catharanthine half-sulfate solution, 200 µl (2.41 µmol) of a 12.0 mM vindoline hydrochloride solution, and 1.0 ml (1.20 mmol) of an aqueous 1.2 M ferric chloride solution were added sequentially in the order mentioned. The mixture was kept cooled with ice and bubbled with nitrogen and, at the same time, stirred for three hours. The mixture was alkalinized by addition of 1 ml of 25% aqueous ammonia and extracted three times with 10 ml of ethyl acetate. The extract consequently collected was dried up under a vacuum, dissolved in 10 ml of water and 24 µl of 2N-HC1, and then stirred in conjunction with 1 ml of an aqueous 227 mM NaBH₄ solution for 30 minutes. Then, the reaction mixture was admixed with 2 ml of 25% aqueous ammonia and extracted three times with 10 ml of ethyl acetate.

The extract collected was dried under a vacuum at a temperature of not higher than 40°C and then analyzed by HPLC under the following conditions. Consequently, 3',4'-anhydrovinblastine was obtained in a yield of 89%.
Column: YMC-packed column, AM-312 (S-5 120A ODS).
Solvent: A 3 : 2 mixture of CH₃CN and an aqueous 0.01 M ammonium carbonate solution.
Flow rate: 1 ml/min.
Column temperature: 45°C
Detection wavelength: 254 nm
Retention time: 3',4'-anhydrovinblastine (40.8 minutes)

### Comparative Example 1

3',4'-Anhydrovinblastine was obtained in a yield of 88% by following the procedure of Example 2, except that 634 mg (3.6 mmol) of L-ascorbic acid was added in place of 1 ml of 25% aqueous ammonia and the resultant mixture and 1 ml (227 µmol) of an aqueous 227 mM NaBH₄ added thereto were stirred for 30 minutes.

### Example 3

3',4'-Anhydrovinblastine was obtained in a yield of 87% by following the procedure of Comparative Example 1, except that triammonium citrate was added in place of L-ascorbic acid.

### Example 4

3',4'-Anhydrovinblastine was obtained in a yield of 85% by following the procedure of Comparative Example 1, except that sodium pyruvate was added in place of L-ascorbic acid.

### Example 5

3',4'-Anhydrovinblastine was obtained in a yield of 87% by following the procedure of Comparative Example 1, except that oxalic acid was added in place of L-ascorbic acid.

### Example 6

3',4'-Anhydrovinblastine was obtained in a yield of 88% by following the procedure of Comparative Example 1, except that malic acid was added in place of L-ascorbic acid.

### Example 7

3',4'-Anhydrovinblastine was obtained in a yield of 84% by following the procedure of Comparative Example 1, except that maleic acid was added in place of L-ascorbic acid.

### Example 8

3',4'-Anhydrovinblastine was obtained in a yield of 85% by following the procedure of Comparative Example 1, except that sodium fluoride was added in place of L-ascorbic acid.

As demonstrated in Examples 1 to 8, this invention allows 3',4'-anhydrovinblastine to be produced in a yield of 89% as compared with the conventional yield of 68.6% (EP-A-282 602). Further, highly pure crystals of 3',4'-anhydrovinblastine can be obtained without requiring separation or purification.

## Claims

1. A method for producing 3',4'-anhydrovinblastine, which comprises reacting catharanthine with vindoline in the presence of Fe³⁺, removing or inactivating the Fe³⁺, by addition of any compound selected from aqueous ammonia, a lower fatty acid, a salt thereof, a dicarboxylic acid, a salt thereof, a ketocarboxylic acid, a salt thereof, a hydroxycarboxylic acid, a salt thereof and a fluoride, and subsequently reacting the reaction product with a reducing agent.

2. A method according to claim 1 wherein Fe³⁺ is inactivated by the addition of an iron ligand.

## Patentansprüche

1. Verfahren zur Herstellung von 3',4'-Anhydrovinplastin, umfassend die Umsetzung von Catharanthin mit Vindolin in Gegenwart von Fe³⁺, Entfernung oder Inaktivierung des Fe³⁺ durch Zugabe irgendeiner Verbindung, ausgewählt aus wäßrigem Ammoniak, einer niederen Fettsäure, einem Salz davon, einer Dicarbonsäure, einem Salz davon, einer Ketocarbonsäure, einem Salz davon, einer Hydroxycarbonsäure, einem Salz davon und einem Fluorid, und anschließende Umsetzung des Reaktionsproduktes mit einem Reduktionsmittel.

2. Verfahren nach Anspruch 1, wobei Fe³⁺ inaktiviert wird durch Zugabe eines Eisenliganden.

## Revendications

1. Procédé de préparation de 3',4'-anhydrovinblastine comprenant la réaction de la catharanthine avec de la vindoline en présence de Fe³⁺, l'élimination ou l'inactivation du Fe³⁺ par addition de n'importe quel composé choisi parmi l'ammoniaque en solution aqueuse, un acide gras, un sel d'un acide gras, un acide dicarboxylique, un sel d'un tel acide, un acide cétocarboxylique, un sel d'un tel acide, un acide hydroxycarboxylique, un sel d'un tel acide et un fluorure, suivie de la réaction du produit réactionnel avec un agent réducteur.

2. Procédé conforme à la revendication 1 dans lequel le Fe³⁺ est inactivé par addition d'un ligand complexant le fer.
